# EUROPEAN PATENT SPECIFICATION

(11) **EP 0 873 735 B1**
(45) Date of publication and mention of the grant of the patent: **28.07.2004**
(21) Application number: 97106949.7
(22) Date of filing: 25.04.1997
(51) Int. Cl.: A61F 9/013

(54) **Automatic microkeratome**
Automatische Mikrokeratomieeinrichtung
Dispositif automatique de microkératomie

(43) Date of publication of application: 28.10.1998
(73) Proprietor: Krumeich, Jörg H., Dr., D-4630 Bochum 6 (DE)
(72) Inventor: Krumeich, Jörg H., Dr., D-4630 Bochum 6 (DE)
(74) Representative: Behrendt, Arne, Dipl.-Ing.

(56) References cited:
- EP-A- 0 442 156
- EP-A- 0 771 553
- WO-A-95/31143
- US-A- 5 586 980

## Description

This invention relates to an apparatus for effecting a planar cutting of the cornea to form a lamella cutting attached by a hinge of corneal tissue to allow treatment by a laser for correction of myopia.

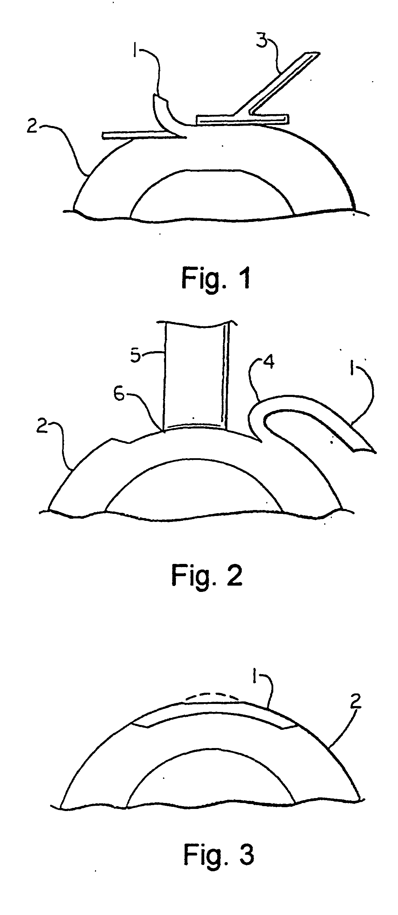

There has been established since about the beginning of 1990 the use of laser treatment for the correction of myopia. When the condition of myopia requires larger corrections to the corneal shape, the principal of LASIK (LASIK= Laser in situ keratomileusis) is employed. As illustrated in Figure 1, this method consists of cutting a thin parallel lamella 1 of corneal tissue from the surface of the cornea 2. To aid in the cutting process a distance holder 3 included in the microkeratome aids in the formation of lamella. The corneal lamella remains attached by a hinged portion 4, as shown in Figure 2, of corneal tissue and thereafter the microkeratome is retracted from the cornea. The flap is then held back and a laser beam 5 is used for treatment of the inside tissue 6 of the cornea. Thereafter as shown in Figure 3 the flap is repositioned on the cornea. The flap is only 13/100 to 18/100 of a mm thick. A suture is not needed as osmotic forces hold the flap in place. The procedure of forming a lamella corneal flap to expose corneal tissue for laser treatment offers the advantage of faster healing, less discomfort for the patient and more stability.

Multiple microkeratomes have been developed over the years, all following the same principal of primarily creating a flat surface on the cornea, thereafter the cut is performed similar to a carpenter's plane. A motor is used to move the blade in all microkeratome and in some microkeratomes rotates the blade.

There is a known microkeratome using one motor for connected movement of both the blade and the microkeratome.

The movement of the microkeratome to execute the corneal cutting is performed on a suction ring that surrounds the corneal circumferences and is adhered firmly to the corneal dome. The position of cornea within the suction ring allows the microkeratome to create a flat corneal surface form the corneal dome. The movement of the microkeratome on the suction ring is guided within dove tail guides. Those kind of apparatuses are embodied for example in EP-A-0 442 156, WO 95 31143 A and US-A-5 586 980.

A disadvantage of these known microkeratomes is that the suction ring, the cutting blade, the guide and the drive mechanism with its gears are parts of a large interconnected structure which is undetachable, openly exposed and therefore apt to interferences like crystalline concrements, conjunctival folds, cilia and as consequence difficult to handle by the physician prior to and during the operation. Further all drive mechanism have tolerances that may change and may influence the precision of the corneal cut.

It is an object of the present invention to provide an apparatus as mentioned above having a detachable suction ring without forfeitures with respect to exact guide and precise control of movement of the microkeratome.

According to the present invention there is provided an apparatus for transversal planar cutting of a cornea to expose a corneal surface to allow correction of myopia as defined in claim 1. The apparatus includes a corneal suction ring, microkeratome guide surfaces extending along opposite sides of an aperture for exposing a portion of cornea, a microkeratome guided by the guide surfaces of the corneal suction ring to execute a transverse planar cut of a cornea surface in the aperture of the cornea suction ring, an electronically controllable actuator controlling relative movement between the corneal suction ring and the microkeratome, a steering rod, operatively interconnecting the electronically controllable actuator and the corneal suction ring and a releasable connection between the suction ring and the steering rod.

To realize the releasable interconnection between the suction ring and the steering rod the suction ring includes a receiver having a cavity for receiving an end portion of the steering rod and a retainer supported by the suction ring.

A further development of the invention provides a second receiver with a retainer on the diametrically opposite site of the suction ring peripheral surface.

The present invention will be more fully understood when the following description is read in light of the accompanying drawings in which:
- Figure 1: is a schematic illustration showing the use of a microkeratome for executing a cut of hinged lamella portion of a cornea to allow laser treatment for correction of myopia;
- Figure 2: is a schematic illustration of the laser correction treatment;
- Figure 3: is a schematic illustration showing the cornea after myopia correction;
- Figure 4: is an elevation view partly in section illustrating a first form of the apparatus for performing the transverse cutting of the cornea according to the present invention;
- Figure 5: is a partial plan view taken along lines 5 - 5 of Figure 4;

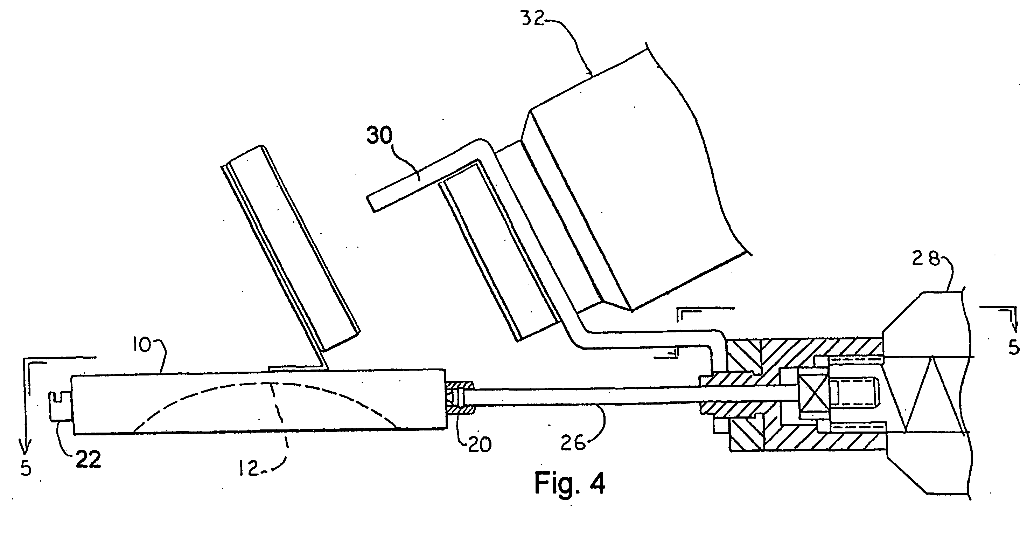

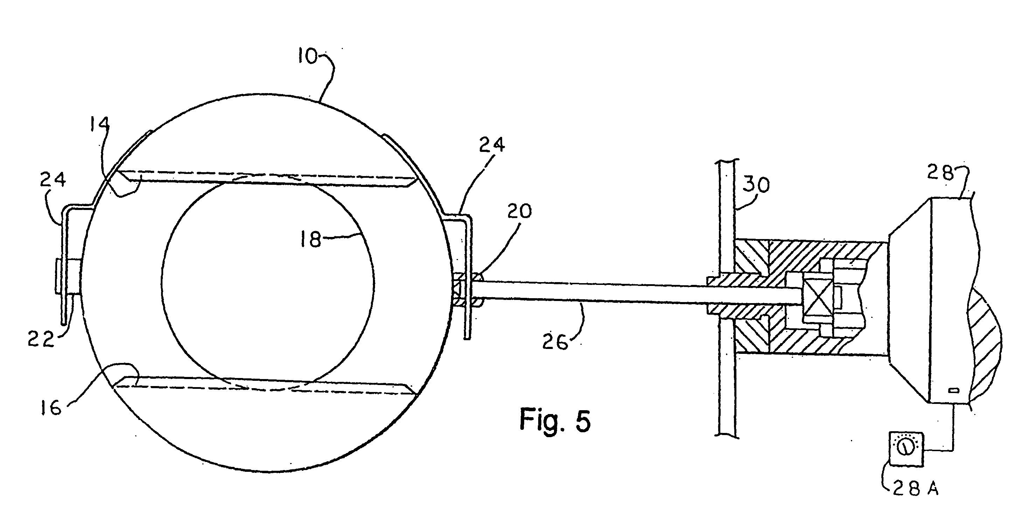

For the purposes of disclosing of a first embodiment of the present invention as shown in Figures 4 and 5 there is illustrated a cornea suction ring 10 having annular side walls and a concave bottom wall 12 having a shape for affixing to the cornea of the eye by suction in a manner as per se well known in the art. On the top surface of the cornea suction ring 10 which is opposite the corneal engaging surface there are formed dove tail guide surfaces 14 and 16 that are spaced apart in parallel extending along opposite sides of an aperture 18 in the suction ring 10. Affixed to the outer peripheral surface of the suction ring 10 at diametrically opposite sites are receiver sockets 20 and 22. The receiver sockets 20 and 22 are centrally spaced between the parallel arrangement of dove tail guide surfaces 14 and 16. Associated with each of the receiver sockets 20 and 22 is a spring clamp 24 which is urged under a resilient force of this spring into a slot traversing the sockets so that a part of clamp 24 protrudes into a hollow pocket of each receiver socket 20 and 22 to engage in an annular recess formed on the end portion of a steering rod 26. The steering rod 26 is connected to a linearly moveable output member of an electronically geared stepping motor 28 that is operated by a controller 28A so that the linear output speed is controllably selected at a given speed usually within the range of 0.5 mm/s to 3 mm/s. Fastened to the housing of stepping motor 28 is an angled bracket 30 which forms a mounting structure for mechanically connecting a microkeratome 32.

The control of the stepping motor 28, in addition to speed, controls the total movement of the microkeratome 32 through establishment of the distance that the steering rod 26 is moved relative to the housing of the stepping motor 28 in this manner, the cut of the corneal tissue by the microkeratome 32 establishes the widths of the hinge attaching the lamella flap to the cornea.

While the present invention has been described in connection only with the preferred embodiments of the various figures, it is to understood that other similar embodiments may be used or modifications or additions may be made to the described embodiments for performing the same function of the present invention without deviating therefrom. Therefore, the present invention should not be limited to a single embodiment, but rather construed in breadth and scope in accordance with the recitation of the appended claims.

## Claims

1. An apparatus for transverse planar cutting of a cornea to expose a corneal surface to allow correction of myopia, said apparatus including:
a) a corneal suction ring (10) including microkeratome guide surfaces (14+16) extending along opposite sides of an aperture for exposing a portion of cornea,
b) a microkeratome (32) guided by the guide surfaces (14+16) of the corneal suction ring (10) to execute at least a partial transverse planar cut of a cornea surface in the aperture of the cornea suction ring (10)
c) and an electronically controllable actuator (28+28A) for controlling relative movement between said corneal suction ring (10) and said microkeratome
**characterized in**
d) a steering rod (26), interconnecting said corneal suction ring (10) and said electronically controllable actuator (28+28A), said actuator (28+28A) displacing the steering rod (26) in the direction in its lenght for the relative movement between the corneal suction ring (10) and the microkeratome,
e) wherein the suction ring (10) includes a receiver (20) having a cavity for receiving an end portion of said steering rod (26) and a retainer (24) supported by the suction ring (10) to maintain a releasable interconnected relation between the steering rod (26) and the suction ring (10).

2. Apparatus according to claim 1, wherein said steering rod extends radially outward from said corneal suction ring in a generally parallel and central relation to the guide surfaces (14 + 16).

3. Apparatus according to claim 1 or 2, wherein the suction ring (10) includes receivers (20 + 22) at diametrically opposite edges of said suction ring (10) with each receiver (20 + 22) being spaced centrally between the guide surfaces (14 + 16), each receiver including a cavity for selectively receiving an and portion of said steering rod (26) and a retainer (24) supported by the suction ring for each receiver (20 + 22) to maintain a releasable connection between said suction ring (10) and said steering rod (26).

## Patentansprüche

1. Eine Vorrichtung zum transversalen, planaren Schneiden einer Kornea, um korneale Oberfläche frei zu legen und die Behandlung von Kurzsichtigkeit zu erlauben, diese Vorrichtung enthaltend:
a) einen kornealen Saugring (10), welcher Mikrokeratom-Führungsflächen (14 + 16) enthält, die sich entlang gegenüberliegender Seiten einer Öffnung zum Freilegen eines Abschnitts der Kornea erstrecken,
b) ein Mikrokeratom (32), welches von den Führungsflächen (14 + 16) des kornealen Saugrings (10) geführt ist, um wenigstens einen Teiltransversalen, planaren Schnitt in die Oberfläche der Kornea innerhalb der Öffnung des kornealen Saugrings (10) auszuführen,
c) und ein elektronisch steuerbares Stellglied (28 + 28A) zum Steuern einer Relativbewegung zwischen dem genannten kornealen Saugring (10) und dem genannten Mikrokeratom,
**gekennzeichnet durch**
d) eine Steuerstange (26), welche den genannten kornealen Saugring (10) und das genannte elektronisch steuerbare Stellglied (28+28A) verbindet, wobei das genannte Stellglied (28 + 28A) die Steuerstange (26) in ihre Längsrichtung für die Relativbewegung zwischen dem kornealen Saugring (10) und dem Mikrokeratom verschiebt,
e) wobei der Saugring (10) eine Aufnahme (20), welche eine Aushöhlung zur Aufnahme eines Endstücks der genannten Steuerstange (26) aufweist und einen Sicherungsring (24) enthält, welcher von dem Saugring (10) abgestützt wird, um eine lösbare Verbindung zwischen der Steuerstange (26) und dem Saugring (10) zu bilden.

2. Vorrichtung nach Anspruch 1, wobei die genannte Steuerstange sich in radialer Richtung auswärts von dem genannten kornealen Saugring in einem im wesentlichen parallelen und mittigen Verhältnis zu den Führungsflächen (14 + 16) erstreckt.

3. Vorrichtung nach Anspruch 1 oder 2, wobei der Saugring (10) Aufnahmen (20 + 22) an gegenüberliegenden Seiten des genannten Saugrings (10) enthält, wobei jede Aufnahme (20 + 22) mittig zwischen den Führungsflächen (14 + 16) angeordnet ist, jede Aufnahme eine Aushöhlung zur wahlweisen Aufnahme eines Endstücks der genannten Steuerstange (26) enthält und wobei der Saugring (10) einen Sicherungsring (24) für jede Aufnahme (20 + 22) enthält, welcher von dem Saugring abgestützt wird, um eine lösbare Verbindung zwischen dem genannten Saugring (10) und der genannten Steuerstange (26) zu bilden.

## Revendications

1. Appareil pour une coupe transversale plane d'une cornée pour exposer une surface de cornée afin de permettre la correction d'une myopie, ledit appareil comportant :
(a) une bague d'aspiration de cornée (10) comportant des surfaces de guidage de microkératome (14+16) s'étendant le long de côtés opposés d'une ouverture pour exposer une portion de la cornée,
(b) un microkératome (32) guidé par les surfaces de guidage (14+16) de la bague d'aspiration de cornée (10) pour exécuter au moins une coupe transversale plane partielle d'une surface de cornée dans l'ouverture de la bague d'aspiration de cornée (10) et
(c) un actionneur (28+28A) à commande électronique pour commander un déplacement relatif entre ladite bague d'aspiration de cornée (10) et ledit microkératome,
**caractérisé en ce qu'**il comporte
(d) une tige de direction (26), interconnectant ladite bague d'aspiration de cornée (10) et ledit actionneur pouvant être commandé électroniquement (28+28A), ledit actionneur (28+28A) déplaçant la tige de direction (26) dans la direction de sa longueur en vue du mouvement relatif entre la bague d'aspiration de cornée (10) et le microkératome,
(e) où la bague d'aspiration (10) comporte un organe de réception (20) présentant une cavité pour recevoir une portion d'extrémité de ladite tige de direction (26) et un organe de retenue (24) supporté par la bague d'aspiration (10) pour maintenir une relation d'interconnexion relâchable entre la tige de direction (26) et la bague d'aspiration (10).

2. Appareil selon la revendication 1, où ladite tige de direction s'étend radialement vers l'extérieur depuis ladite bague d'aspiration de cornée en une relation généralement parallèle et centrale aux surfaces du guidage (14 + 16).

3. Appareil selon la revendication 1 ou 2, où la bague d'aspiration (10) comporte des organes de réception (20+22) à des bord diamétralement opposés de ladite bague d'aspiration (10), chaque organe de réception (20+22) étant espacé centralement entre les surfaces de guidage (14+16), chaque organe de réception présentant une cavité pour recevoir sélectivement une portion d'extrémité de ladite tige de direction (26) et un organe de retenue (24) supporté par la bague d'aspiration pour chaque organe de réception (20+22) pour maintenir une connexion relâchable entre ladite bague d'aspiration (10) et ladite tige de direction (26).
